(19) Europäisches Patentamt / European Patent Office / Office européen des brevets

(11) **EP 3 831 904 B1**

(12) **EUROPEAN PATENT SPECIFICATION**

(45) Date of publication and mention of the grant of the patent:
**18.12.2024 Bulletin 2024/51**

(21) Application number: **20786430.7**

(22) Date of filing: **11.06.2020**

(51) International Patent Classification (IPC):
**C09J 11/04** *(2006.01)*   **C09J 153/02** *(2006.01)*
**C09J 201/00** *(2006.01)*   **A61F 13/15** *(2006.01)*
**A61F 13/51** *(2006.01)*   **A61L 9/00** *(2006.01)*
**A61Q 19/00** *(2006.01)*   **C08J 3/22** *(2006.01)*
**A61K 8/27** *(2006.01)*   **C09J 11/06** *(2006.01)*
**C08K 9/02** *(2006.01)*   **C08K 7/26** *(2006.01)*
**C08K 5/00** *(2006.01)*

(52) Cooperative Patent Classification (CPC):
(C-Sets available)
**C09J 11/06; A61F 13/15; C08J 3/22; C09J 153/02;**
A61F 2013/51076; C08K 3/11; C08K 5/0058;
C08K 7/26; C08K 9/02                              (Cont.)

(86) International application number:
**PCT/JP2020/023079**

(87) International publication number:
**WO 2021/070413 (15.04.2021 Gazette 2021/15)**

(54) **PRESSURE-SENSITIVE ADHESIVE COMPOSITION, METHOD FOR PRODUCING PRESSURE-SENSITIVE ADHESIVE COMPOSITION, AND ARTICLE INCLUDING PRESSURE-SENSITIVE ADHESIVE COMPOSITION**

DRUCKEMPFINDLICHE KLEBSTOFFZUSAMMENSETZUNG, VERFAHREN ZUR HERSTELLUNG EINER DRUCKEMPFINDLICHEN KLEBSTOFFZUSAMMENSETZUNG UND ARTIKEL MIT DRUCKEMPFINDLICHER KLEBSTOFFZUSAMMENSETZUNG

COMPOSITION D'ADHÉSIF SENSIBLE À LA PRESSION, PROCÉDÉ DE PRODUCTION D'UNE COMPOSITION ADHÉSIVE SENSIBLE À LA PRESSION, ET ARTICLE CONTENANT UNE COMPOSITION ADHÉSIVE SENSIBLE À LA PRESSION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.10.2019 JP 2019185099**

(43) Date of publication of application:
**09.06.2021 Bulletin 2021/23**

(73) Proprietor: **Koyo Corporation**
**Yokohama-shi, Kanagawa 236-0004 (JP)**

(72) Inventors:
• **SHIRAI, Atsuko**
  **Yokohama-shi, Kanagawa 2360004 (JP)**

• **IKEDA, Ryuzo**
  **Sashima-gun, Ibaraki 3060313 (JP)**
• **KAKINUMA, Hideyuki**
  **Sashima-gun, Ibaraki 3060313 (JP)**

(74) Representative: **Gulde & Partner**
**Patent- und Rechtsanwaltskanzlei mbB**
**Wallstraße 58/59**
**10179 Berlin (DE)**

(56) References cited:
**EP-A2- 0 697 212      WO-A1-2007/099738**
**WO-A1-2018/131713      JP-A- 2005 095 203**
**JP-A- 2007 246 797      JP-A- 2008 038 021**
**JP-A- 2008 038 021      JP-A- 2017 176 757**
**JP-A- 2018 114 030      JP-A- H08 207 561**
**US-A1- 2014 331 601**

(52) Cooperative Patent Classification (CPC): (Cont.)

C-Sets
**C09J 153/02, C08K 3/11;**
**C09J 153/02, C08K 5/0058;**
**C09J 153/02, C08K 9/02**

**Description**

**Technical Field**

The present invention relates to an adhesive composition, a method for producing the adhesive composition and an object using the adhesive composition.

**Background Art**

Various forms of deodorants have been proposed so far in order to effectively remove malodors in the living space. Among them, an adhesive composition containing a deodorant is useful because it has both an object adhering function and a deodorizing function (JP 6294978 B).

WO 2018/131713 A1 discloses an adhesive composition comprising a porous deodorant, such as zinc ion-supporting zeolite, and an adhesive base.

WO 2007/099738 A1 discloses a deodorant cosmetic composition, such as a deodorant stick, comprising an antibacterial zeolite and an oil-absorbing powder. The antibacterial zeolite comprises metals ions selected from silver, copper and zinc ions.

EP 0 697 212 A2 also relates to deodorant cosmetic compositions comprising a zeolite having at least a part of its exchangeable ions substituted with ammonium ions and antibacterial metal ions selected from silver, copper, zinc, mercury, tin, lead, bismuth, cadmium, chromium, and thallium.

**Summary of Invention**

**Technical Problem**

On the other hand, from the viewpoint of hygiene, it is required to have an antibacterial function in addition to the deodorizing function. However, since antibacterial agents using silver or the like are generally expensive, it is required to reduce the amount used.

The present invention has been made in view of such problems, and provides an adhesive composition having excellent deodorizing effect and antibacterial effect while suppressing the amount of expensive antibacterial agent used.

**Solution to Problem**

According to the present invention, there is provided an adhesive composition as defined in claim 1. The adhesive composition comprising a porous deodorant containing a zinc ion-supporting zeolite, an antibacterial agent containing a silver ion-supporting zeolite, and an adhesive base, wherein the content of the porous deodorant is 0.25 mass% or more, and the content of the antibacterial agent is 0.1 mass% or more based on the total amount of the adhesive composition.

As a result of intensive studies, the present inventors have found that the deodorizing effect and antibacterial effect are excellent when a predetermined amount or more of said antibacterial agent and said porous deodorant are used in combination, and have completed the present invention. That is, it has been found that at least the antibacterial effect is greater than the effect expected according to each effect of the deodorant and the antibacterial.

Various embodiments of the present invention are exemplified below. Embodiments shown below may be combined with each other.

Preferably, the content of the porous deodorant is 0.4mass% or more, and the content of the antibacterial agent is 0.15 - 0.5mass%.

Preferably, the adhesive composition has a softening point higher than 70°C.

Preferably, the adhesive base contains one or more thermoplastic elastomers from the group consisting of a styrene-isoprene-styrene block polymer, a styrene-butadiene-styrene block copolymer, a styrene-ethylene-butylene-styrene block copolymer, and a styrene-ethylene-propylene-styrene block copolymer.

According to another aspect of the present invention, there is provided a method for producing the adhesive composition, which comprises a process where a masterbatch containing the porous deodorant and the antibacterial agent is mixed with an adhesive base component other than the adhesive base component contained in the masterbatch; or a process where a masterbatch containing the porous deodorant and a masterbatch containing the antibacterial agent are mixed with an adhesive base component other than the adhesive base component contained in the masterbatches.

According to another aspect of the invention, there is provided an object comprising a laminated structure part in which at least two layers are adhered to each other by the adhesive composition.

Preferably, the object is a body fluid absorbing object having the laminated structure part.

**Brief Description of Drawings**

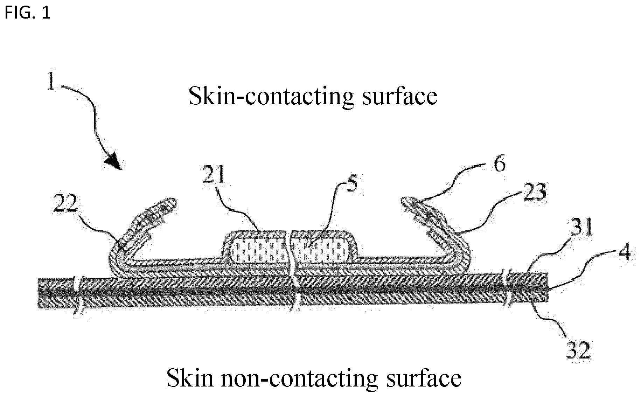

FIG. 1 is a schematic cross-sectional view showing a preferred form of a body fluid absorbing object using an adhesive composition.

**Description of Embodiments**

Hereinafter, various embodiments of the present invention will be described. Various distinctive features shown in the following embodiments can be combined with each other. In addition, an invention can be established independently for each of the distinctive features.

1. Adhesive Composition

The adhesive composition according to an embodiment of the present invention comprises a porous deodorant, an antibacterial agent and an adhesive base.

(Porous Deodorant)

The porous deodorant may be obtained by granulating and molding zeolite.

As the porous deodorant, zeolite supporting zinc ions is used (hereinafter referred to as zinc ion-supporting zeolite). On the other hand, it is preferable not to contain silver from the viewpoint of cost. By using the zinc ion-supporting zeolite as the deodorant, discoloration due to heating or a chemical reaction with the adhesive base during production does not occur, and thus it is possible to provide an adhesive composition which is not colored. In addition, as one embodiment of the zeolite supporting metal ions, there is a zeolite in which some or all of the ion-exchangeable ions in the zeolite are replaced with metal ions.

Further, the porous deodorant is preferably one which exerts the deodorizing effect by adsorbing and decomposing any or all of ammonia, hydrogen sulfide, a mercaptan compound and the like.

In addition, it is preferable that the porous deodorant has a white or light color from the viewpoint of commercialization.

Further, the lower limit of the average particle size of the porous deodorant can be set to preferably 0.5 $\mu$m or more, more preferably 1.0 $\mu$m or more. When the porous deodorant has an average particle size of not less than the lower limit, aggregation of the porous deodorant in the adhesive composition is less likely to occur.

The upper limit of the average particle size of the porous deodorant is preferably 300 $\mu$m or less, more preferably 100 $\mu$m or less, and further preferably 50 $\mu$m or less. When the porous deodorant has an average particle size of not more than the upper limit, the deodorant in the adhesive composition is less likely to adhere and precipitate in the nozzle or hose and the dust filter of the coating device. That is, since the transport loss of the deodorant is reduced in the coating device, a fixed amount of the deodorant can be surely applied, and the effort and cost for removing (cleaning) the deodorant inside the device can be reduced. The average particle size of the porous deodorant can be measured in accordance with, for example, a laser diffraction-type particle size distribution measuring method.

From the viewpoints of deodorizing effect and antibacterial effect, the content of the porous deodorant is 0.25 mass% or more, and preferably 0.4 mass% or more, based on the total amount of the adhesive composition.

Further, from the viewpoints of coating property and adhesive property, the content of the porous deodorant is preferably 20 mass% or less based on the total amount of the adhesive composition. From the viewpoint of cost and the like, the content of the porous deodorant is preferably 3.0 mass% or less, more preferably 1.0 mass% or less, further preferably 0.8 mass% or less, in consideration of and the like.

(Antibacterial Agent)

The antibacterial agent has an antibacterial function and contains silver from the viewpoints of antibacterial effect, low toxicity to human body, and easy availability. Further, according to the invention a zeolite supporting silver ions is used. By using the zeolite, it can be expected to have a deodorizing effect in addition to the antibacterial effect. In addition, as one embodiment of the zeolite supporting silver ions, there is a zeolite in which some or all of the ion-exchangeable ions in the zeolite are replaced with silver ions.

Further, the antibacterial agent has an antibacterial effect against Staphylococcus aureus and the like.

The lower limit of the average particle size of the antibacterial agent used in the present invention can be set to preferably 0.5 $\mu$m or more, more preferably 1.0 $\mu$m or more. When the antibacterial agent has an average particle size of not less than the lower limit, aggregation of the antibacterial agent in the adhesive composition is less likely to occur.

The upper limit of the average particle size of the antibacterial agent used in the present invention is preferably 300 µm or less, more preferably 100 µm or less, and further preferably 50 µm or less. When the antibacterial agent has an average particle size of not more than the upper limit, the antibacterial agent in the adhesive composition is less likely to adhere and precipitate in the nozzle or hose and the dust filter of the coating device. That is, since the transport loss of the antibacterial agent is reduced in the coating device, a fixed amount of the antibacterial agent can be surely applied, and the effort and cost for removing (cleaning) the antibacterial agent that has adhered and precipitated inside the device can be reduced. The average particle size of the antibacterial agent can be measured in accordance with, for example, a laser diffraction-type particle size distribution measuring method.

The content of the antibacterial agent is 0.1 mass% or more, and preferably 0.15 to 0.5 mass%, based on the total amount of the adhesive composition, from the viewpoints of deodorizing effect, antibacterial effect, cost, and the like.

(Adhesive Base)

The adhesive base is not particularly limited as long as it is a base for various adhesive compositions.

For example, when the adhesive base contains a vinyl acetate resin, a plasticizer, a preservative, a surfactant, etc., the adhesive composition can be a vinyl acetate resin-based emulsion-type adhesive.

When the adhesive base contains isobutene, a maleic anhydride copolymer resin solvent, an SBR latex aqueous epoxy compound, etc., the adhesive composition can be an α-olefin adhesive.

When the adhesive base contains an acrylic copolymer resin, a plasticizer, a filler, etc., the adhesive composition can be an acrylic resin-based emulsion-type adhesive.

When the adhesive base contains a vinyl acetate resin, an ethyl acetate, a methanol, etc., the adhesive composition can be a vinyl acetate resin-based solvent-type adhesive.

When the adhesive base contains an acrylic copolymer resin, a tackifying resin, a plasticizer, a crosslinking agent, a filler, etc., the adhesive composition can be an acrylic resin-based solvent-type adhesive.

When the adhesive base contains a chloroprene rubber, a solvent, a tackifying resin, a filler, etc., the adhesive composition can be a chloroprene rubber-based solvent-type mastic adhesive.

When the adhesive base contains isocyanate/polyol, a resin, a filler, etc., the adhesive composition can be a urethane resin-based adhesive.

When the adhesive base contains a modified silicone resin, a filler, a plasticizer, a silane coupling agent, etc., the adhesive composition can be a modified silicone resin-based adhesive.

When the adhesive base contains starch, water, a bulking agent, a stabilizer, a preservative, a fungicide, etc., the adhesive composition can be a starch-based adhesive.

When the adhesive base contains a polymer admixture (acrylic emulsion, SBR latex, etc.), cement, fine aggregate, etc., the adhesive composition can be a polymer cement mortar.

When the adhesive base contains a silylated urethane resin, a filler, a plasticizer, a silane coupling agent, etc., the adhesive composition can be a silylated urethane resin-based adhesive.

When the adhesive base contains ethyl vinyl alcohol or a thermoplastic elastomer, a softening agent, a tackifying resin, etc., the adhesive composition can be a hot melt adhesive.

When the adhesive composition is a hot melt adhesive, examples of the thermoplastic elastomer include styrene-isoprene-styrene block copolymer (SIS), styrene-butadiene-styrene block copolymer (SBS), styrene-ethylene-butylene-block copolymer (SEBS), styrene-ethylene-propylene-styrene block copolymer (SEPS), olefin-based thermoplastic elastomer, polybutadiene-based thermoplastic elastomer, polyurethane-based thermoplastic elastomer, polyester-based thermoplastic elastomer, polyamide-based thermoplastic elastomer, vinyl chloride-based thermoplastic elastomer, polyester-based thermoplastic elastomer.

Among them, styrene-isoprene-styrene block copolymer (SIS), styrene-butadiene-styrene block copolymer (SBS), styrene-ethylene-butylene-block copolymer (SEBS), styrene-ethylene-propylene-styrene block copolymer (SEPS) are preferable as the thermoplastic elastomer.

The adhesive base may contain these thermoplastic elastomers alone or may contain two or more kinds in combination.

By using these thermoplastic elastomers, an adhesive composition having high adhesive strength can be produced.

From the viewpoint of coating property, the content of the thermoplastic elastomer is preferably 50 mass% or less, more preferably 30 mass% or less, and further preferably 25 mass% or less, based on the total amount of the adhesive composition.

Further, from the viewpoints of adhesive property and creep characteristics (especially creep characteristics under high temperature environment (eg, 50°C.)), the content of the thermoplastic elastomer is preferably 1 mass% or more, more preferably 5 mass% or more, further preferably 10 mass% or more, further preferably 12 mass% or more, further preferably 12.5 mass% or more.

When the content of the thermoplastic elastomer is in the above range, and the content of the porous granular deodorant is in the above-mentioned preferable range, it is possible to provide an adhesive composition having excellent deodorizing effect, adhesive property and coating property.

When the adhesive composition is in the form of a hot melt adhesive, adhesive base preferably contains a tackifying resin.

The tackifying resin is not particularly limited as long as it is a resin that improves the adhesive properties of the adhesive composition, but is preferably has a light color or no color. It is more preferable that the tackifying resin has no odor. Further, a tackifying resin that is not deteriorated by heating during production and use of the deodorant adhesive composition is more preferable.

The tackifying resin that can be used in the present invention is not particularly limited, and examples thereof include natural rosins such as gum rosin, wood rosin, tall oil rosin, modified rosins such as distilled rosin, hydrogenated rosin, dimerized rosin and polymerized rosin, glycerol esters such as glycerol ester of off-white wood rosin, glycerol ester of hydrogenated rosin, glycerol ester of polymerized rosin, pentaerythritol esters such as pentaerythritol ester of off-white wood rosin, pentaerythritol ester of hydrogenated rosin, pentaerythritol ester of tall oil rosin, and phenol-modified pentaerythritol ester of rosin, polyterpene resins, copolymers and terpolymers such as styrene/terpene, $\alpha$-methylstyrene/terpene, vinyltoluene/terpene, phenol-modified terpene resins, aliphatic petroleum hydrocarbon resins, hydrogenated petroleum resins, aromatic petroleum resins and the like.

These tackifying resins may be contained in the base alone or in combination of two or more.

The content of the tackifying resin is not particularly limited, but can be , for example, 20 to 90 mass%, more preferably 40 to 80 mass%, further preferably 50 to 70 mass%, based on the total amount of the adhesive composition.

When the adhesive composition is a hot melt adhesive, the adhesive base preferably contains a softening agent.

Examples of the softening agent include petroleum-based process oils such as paraffin-based process oil, naphthene-based process oil and aroma-based process oil, olefin oligomer and low molecular weight polymer, vegetable oil, animal oil, and derivatives thereof.

These softening agents may be contained in the base alone or in combination of two or more.

The content of the softening agent is not particularly limited, but can be, for example, preferably 5 to 60 mass%, more preferably 10 to 40 mass%, and further preferably 15 to 25 mass%, based on the total amount of the adhesive composition.

It is preferable that the softening agent has an achromatic color from the viewpoint of commercialization.

In addition to the above-mentioned adhesive base, the adhesive base may contain optional components as long as the effect of the present invention is not impaired. For example, examples of the said optional components include a stabilizer, an antioxidant, a leveling agent, a preservative, a defoamer, a thickener, a rheology modifier, inorganic fillers such as silica, alumina, boron nitride, glass beads, a flame retardant, or a colorant, a solvent and the like.

The content of the optional component is not particularly limited as long as the effect of the present invention is not impaired, but can be, for example, 0.1 to 2 mass%, preferably 0.1 to 1 mass%.

(Characteristics of Adhesive Composition)

From the viewpoint of coating properties, the adhesive composition preferably has a viscosity at 160°C and 1 atm of 10000 mPa·s or less, more preferably 8000 mPa·s or less, more preferably 5000 mPa·s or less.

From the viewpoint of adhesive property, the adhesive composition preferably has a viscosity at 160°C and 1 atm of 1000 mPa·s or more, more preferably 2000 Pa s or more.

The viscosity of the adhesive composition can be measured by a Brookfield viscometer equipped with a heating chamber described in JIS K6862-1984.

The viscosity of the adhesive composition can be adjusted by changing the content of the porous granular deodorant and the composition of the adhesive base. For example, when a tackifier, a softening agent, and a thermoplastic elastomer is used in the adhesive base, it can be adjusted to a desired viscosity by increasing or decreasing the content of the thermoplastic elastomer.

From the viewpoint of coating property, the softening point of the adhesive composition is preferably higher than 70°C, more preferably higher than 73°C. The softening point of the adhesive composition can also be adjusted by changing the content of the porous deodorant or the composition of the adhesive base.

The coating property means the ease of coating, and particularly the coating applicability by a coating device. Examples of such coating devices include a coating device capable of spray coating or curtain coating (for example, Nordson's non-woven & coating system).

Further, the adhesive property means the difficulty of peeling.

For example, it can be said that the adhesive composition has adhesive property when the peel strength at room temperature is high. Specifically, it can be said that the adhesive composition has adhesive property when the peel strength is preferably 5 N/25 mm or more, more preferably 10 N/25 mm or more.

Examples of the method of measuring the peel strength include a method of measuring by a 180 degree peel test and a method of measuring by a T-type peel test.

It can also be said that the adhesive composition has adhesive property when there is sufficient resistance when the adhered test piece that has been left standing at a low temperature (for example, -10°C) for a certain period of time is taken out and immediately peeled off at high speed (low-temperature manual peeling).

Furthermore, the adhesive property of the adhesive composition can also be evaluated by combining the two evaluations of normal temperature peel strength and low temperature manual peeling.

2. Method for Producing Adhesive Composition

The adhesive composition can be produced by mixing a porous deodorant and an antibacterial agent with an adhesive base by a conventional method, but it is preferably produced by mixing a pre-prepared masterbatch containing a porous deodorant and an antibacterial agent with the remaining adhesive base component. That is, the method for producing the adhesive composition according to one embodiment of the present invention comprises a process where a masterbatch containing a porous deodorant and an antibacterial agent is mixed with an adhesive base component other than the adhesive base component contained in the masterbatch. Thus, it is possible to produce an adhesive composition exhibits a high deodorizing effect. In addition, a masterbatch containing a porous deodorant and a masterbatch containing an antibacterial agent may be prepared respectively to use two masterbatches, or a masterbatch containing both a porous deodorant and an antibacterial agent may be prepared and used.

The masterbatch can be prepared, for example, by heating and mixing a part of the adhesive base, the porous deodorant and/or the antibacterial agent in a mixing pot.

The content of each of the porous deodorant and the antibacterial agent in the masterbatch is, for example, preferably 20 to 80 mass%, more preferably 30 to 60 mass%, and further preferably 35 to 45 mass%.

When the content of the masterbatch is within the above range, the porous deodorant and the antibacterial agent can be more uniformly dispersed, and an adhesive composition excellent in deodorizing effect and antibacterial effect can be produced.

In the case of producing a hot melt adhesive, as the adhesive base, a tackifying resin, a softening agent, and, if necessary, an antioxidant are added to the masterbatch.

In this case, the content of the tackifying resin in the masterbatch is, for example, 20 to 80 mass%, more preferably 30 to 60 mass%, and further preferably 35 to 45 mass%.

The content of the softening agent in the masterbatch is, for example, 5 to 40 mass%, more preferably 10 to 30 mass%, and further preferably 15 to 25 mass%.

The method of mixing the masterbatch and the remaining adhesive base component can be carried out by a conventional method. For example, a method of adding the masterbatch thereto while mixing the adhesive base in a mixing pot can be applied.

3. Object Using Adhesive Composition

An object coated with the adhesive composition of the present invention has excellent deodorizing and antibacterial effects. More specifically, it is preferable that the above-mentioned adhesive composition is applied to adhering members to be joined together and an object is formed.

There is no limitation on the object to which the adhesive composition of the present invention can be applied, but it is preferably applied to an object having two or more layers adhered to each other. If adhering between layers in the object is realized by the adhesive composition of the present invention, it is possible to manufacture the object and impart the deodorizing effect to the object at the same time.

That is, the present invention also relates to an object having a laminated structure in which at least two layers are adhered to each other with the adhesive composition described above.

Examples of the object having such a laminated structure include body fluid absorbing objects such as paper diapers, napkins, panty liners, incontinence pads, toilet sheets for pets, disposable wear objects used during surgery such as gowns, masks, packaging materials, filters, sound absorbing materials, sound insulating materials, heat insulating materials, oil adsorbing materials, insulating separators and the like.

Among them, the body fluid absorbing object has a problem in deodorizing the odor derived from urine and blood, and thus the present invention can be preferably applied.

Hereinafter, an embodiment in which the present invention is applied to a paper diaper among the body fluid absorbing object will be described in detail with reference to the schematic sectional view of FIG. 1.

The paper diaper 1 comprises a liquid-permeable sheet 21, a liquid non-permeable sheet 22 and an absorber 5 between these two sheets on the skin-contacting surface. The absorber 5 is not particularly limited in its material and shape as long as it can absorb a liquid, and examples thereof include a non-woven fabric, a tissue, a fluff, and the like, and may include a super absorbent resins (SAP) and the like.

The liquid-permeable sheet 21 is preferably formed of, for example, fabrics, films, or a laminated body therof. Examples of the fabrics and films include a thermal bonded nonwoven fabric, a spunlace nonwoven fabric, a point-bonded nonwoven fabric, a spunbonded nonwoven fabric, and a perforated film. Further, it is more preferable that the liquid-permeable sheet 21 has its skin-contacting surface subjected to hydrophilic treatment.

The liquid non-permeable sheet 22 is preferably a thermoplastic resin film, a spunlace nonwoven fabric made of thermoplastic fiber, a point-bonded nonwoven fabric, a spunbonded nonwoven fabric, a meltblown nonwoven fabric, or a laminated body thereof, which has water repellency.

It is more preferable that the paper diaper 1 comprises a side sheet 23 which has a rubber thread 6 fixed to the end part thereof and has elasticity. By providing the side sheet 23, the paper diaper 1 is in close contact with the skin when worn, and thus liquid leakage can be prevented.

The side sheet 23 can be composed of an air-through nonwoven fabric, a spunbonded nonwoven fabric, a meltblown nonwoven fabric, a film, a perforated film, or the like.

The paper diaper 1 comprises an exterior liquid non-permeable sheet 31 and an exterior sheet 32 on the skin non-contacting surface side. And these two sheets are joined by the adhesive composition 4 of the present invention.

The exterior liquid non-permeable sheet 31 is preferably made of, for example, a polyolefin such as polyethylene or polypropylene-based resin sheet.

The exterior sheet 32 is preferably made of a non-woven fabric.

Since the paper diaper 1 has a structure in which the laminated structure part (the exterior liquid non-permeable sheet 31 and the exterior sheet 32) constituting the crotch part is adhered by the adhesive composition 4 of the present invention, it is possible to effectively suppress the odor derived from the absorbed body fluid such as urine.

The adhesive composition 4 of the present invention may be used for adhering the liquid-permeable sheet 21, the liquid non-permeable sheet 22, and the side sheet 23 together.

Further, when the present invention is applied to a body fluid absorbing object having a structure other than the structure shown in FIG. 1, the coating site of the adhesive composition of the present invention can be appropriately designed according to the structure.

It is also preferable to apply the adhesive composition of the present invention to the adhering of fibrous sheets such as non-woven fabric.

The method of coating the adhesive composition is not particularly limited, and examples thereof include non-contact coating and contact coating, but non-contact coating such as spray coating and curtain coating is more preferable.

As a fluid transfer method, a positive displacement method is generally used, and a method using a reciprocating pump (piston pump, plunger pump, etc.) or a rotary pump (gear pump, vane pump, etc.) can be mentioned.

When coating the adhesive composition, a coating method is not limited. For example, as the non-contact coating method, the adhesive composition may be coated in coating methods such as spray-spiral coating, mini spray-mini spiral coating, or fiber-curtain coating.

Among them, from the viewpoints of antibacterial effect and deodorizing efficiency, it is preferable to coat the adhesive composition of the present invention by mini spray-mini spiral coating or fiber-curtain coating, which enables uniform coating even with a small amount.

**EXAMPLES**

Hereinafter, the present invention will be described in more detail with reference to examples. Further, these are merely examples, and do not limit the content of the present invention.

(Production of Adhesive Composition)

A hot melt adhesive was produced by the following method using a zinc ion-supported zeolite deodorant (average particle size: 1.5 $\mu$m; Dushlite ZH10N: manufactured by Sinanen Zeomic Co., Ltd.) as the porous deodorant, a silver ion-supported zeolite antibacterial agent as the antibacterial agent (average particle size:2.5 $\mu$ m,HW10N:manufactured by Sinanen Zeomic Co., Ltd.), and a styrene-butadiene-styrene block copolymer (TR2827:manufactured by JSR Corporation), a hydrogenated petroleum resin (Arkon M100: manufactured by Arakawa Chemical Industries Co., Ltd.), a process oil (SUNPURE NX90: manufactured by Japan Sun Oil Co.Ltd.), a phenol-based antioxidant (Irganox 1010: manufactured by BASF), a phosphorus-based antioxidant (Irgafos 168: manufactured by BASF) as the adhesive base.

First, 40 mass% of the porous deodorant, 42 mass% of the hydrogenated petroleum resin, 18 mass% of the process oil, 0.2 mass% of the phenol-based antioxidant, and 0.2 mass% of the phosphorus-based antioxidant were mixed at 140°C for 1 h to produce a deodorant masterbatch.

In the same manner, 40 mass% of the antibacterial agent, 42 mass% of the hydrogenated petroleum resin, 18 mass% of the process oil, 0.2 mass% of the phenol-based antioxidant, and 0.2 mass% of the phosphorus-based antioxidant were mixed to produce an antibacterial masterbatch.

Next, according to the composition (mass%) shown in Table 1, the two types of the masterbatches were mixed with the remaining adhesive base components to produce the adhesive composition of Example 1. Example 2 and Comparative Examples 1 to 6 were manufactured in the same manner.

(Measurement of Deodorizing Effect)

The adhesive compositions of Examples 1 and 2 and Comparative Examples 1 to 6 were coated on a PET film with a coating film thickness of 5 $\mu$m and a coating area of 100 cm$^2$ (5×20), and this was placed in a 5-liter bag in the environment of a gas concentration of 10 ppm for each gas (ammonia and hydrogen sulfide). After a certain period of time, the residual concentration of the gas in the bag was measured in accordance with JIS K0804-1998 using a detector tube type gas measuring instrument. In the measurement, a blank test in which only ammonia was enclosed in the bag was also performed. The results are shown in Table 1.

The reduction rate is calculated by the following formula when the residual concentration in the bag of the blank test is set to A (ppm) and the residual concentration in the bag of each sample is set to B (ppm).

$$\text{Reduction rate (\%)} = 100 \times (A-B)/A$$

(Measurement of Antibacterial Effect)

The adhesive compositions of Examples 1 and 2 and Comparative Examples 1 to 6 were cultured in accordance with the test method of JIS L1902, and the Staphylococcus aureus count after culturing was measured. The results are shown in Table 1. "E" such as E2 to E4 means an index, for example, "E5=$1\times10^5$ to $9\times10^5$". Further, "ND" means that it was not detected. Then, ND and E2 were evaluated as A, E3 was evaluated as B, and E4 and E5 were evaluated as C.

(Viscosity of Adhesive Composition)

The viscosity of the adhesive composition was measured at 160°C using a Brookfield viscometer equipped with a heating chamber in accordance with JIS K6862-1984. The results are shown in Table 1.

(Softening Point)

The softening point of the adhesive composition was measured by the ring and ball method in accordance with JAI 7-1999 (Japan Adhesive Industry Standard). The results are shown in Table 1.

Further, adhesive property of the adhesive composition was evaluated based on the measured peel strength and the result of low-temperature manual peeling. The results are shown in Table 1.

(Peel Strength)

The peel strength of the adhesive composition was measured by the following method. In accordance with JIS Z0237-2009, the adhesive composition was coated to a support, 38 micron corona-treated PET film, in a thickness of 50 $\mu$m, and the PET film having an untreated surface was pasted to prepare a test piece. Then, the test piece was left standing for a predetermined time and then the force when peeled off at a crosshead speed of 100 mm/min was measured. The results are shown in Table 1.

(Low-Temperature Manual Peeling)

The evaluation by low-temperature manual peeling was performed by storing the test piece under the same conditions as described above for 1 h in a constant temperature bath at -10°C, and then taking it out and immediately peeling it at high speed and observing the degree of resistance.

When the resistance of the high-speed manual peeling was equivalent to the resistance of peeling a control test piece with commercially available polypropylene-based acrylic adhesive tape attached to an aluminum plate at high speed at room temperature, it was evaluated as good. When the resistance was insufficient, it was evaluated as poor. The results are shown in Table 1.

| Table 1 | | | Ex. 1 | Ex. 2 | C.E. 1 | C.E. 2 | C.E. 3 | C.E. 4 | C.E. 5 | C.E. 6 |
|---|---|---|---|---|---|---|---|---|---|---|
| Composition | Block copolymer | | 23 | 23 | 23 | 23 | 23 | 23 | 23 | 23 |
| | Hydrogenated petroleum resin | | 56 | 56 | 56 | 56 | 56 | 56 | 56 | 56 |
| | Process oil | | 20 | 20 | 20 | 20 | 20 | 20 | 20 | 20 |
| | Phenol-based antioxidant | | 0.2 | 0.2 | 0.2 | 0.2 | 0.2 | 0.2 | 0.2 | 0.2 |
| | Phosphorus-based antioxidant | | 0.2 | 0.2 | 0.2 | 0.2 | 0.2 | 0.2 | 0.2 | 0.2 |
| | Porous deodorant | | 0.4 | 0.4 | 0 | 0.4 | 0.6 | 0.2 | 0 | 0 |
| | Antibacterial agent | | 0.2 | 0.4 | 0.2 | 0 | 0 | 0.2 | 0 | 0.4 |
| Evaluation | Viscosity at 160°C(mPa · s) | | 3620 | 3620 | 3600 | 3620 | 3650 | 3600 | 3600 | 3600 |
| | Softening point(°C) | | 82 | 82 | 81 | 81 | 82 | 81 | 80 | 81 |
| | Adhesive property | Peel strength(N/25mm) | 12 | 12 | 12 | 12 | 12 | 12 | 12 | 12 |
| | | Low-Temperature Manual Peeling | Good | Good | Good | Good | Good | Good | Good | Good |
| | Deodorizing effect | Residual ammonia concentration after 2 h (ppm) | 4 | 4 | 7 | 5 | 1 | 6 | 8 | 7 |
| | | Ammonia concentration reduction rate after 2 h (%) | 60% | 60% | 30% | 50% | 90% | 40% | 20% | 30% |
| | | Residual ammonia concentration after 24 h (ppm) | 2 | 2 | 4 | 2.5 | 0 | 3 | 5 | 4 |
| | | Ammonia concentration reduction rate after 24 h (%) | 80% | 80% | 60% | 75% | 100% | 70% | 50% | 60% |
| | | Residual hydrogen sulfide concentration after 2 h (ppm) | 4 | 3.9 | 4 | 3.8 | 3 | 4 | 4 | 4 |
| | | Reduction rate of hydrogen sulfide concentration after 2 h (%) | 0 | 3% | 0% | 5% | 25% | 0% | 0% | 0% |
| | | Residual hydrogen sulfide concentration after 24 h (ppm) | 2.1 | 1.8 | 3 | 2.2 | 0 | 2.5 | 4 | 2.8 |
| | | Reduction rate of hydrogen sulfide concentration after 24 h(%) | 48% | 55% | 25% | 45% | 100% | 38% | 0% | 30% |
| | Antibacterial effect | Staphylococcus aureus count after culturing | E2 | ND | E4 | E5 | E5 | E3 | E5 | E2 |
| | | Antibacterial activity judgment | A | A | C | C | C | B | C | A |
| Ex. denotes "Example" C.E. denotes "Comparative Example" | | | | | | | | | | |

### Claims

1. An adhesive composition comprising:

   a porous deodorant containing a zinc ion-supporting zeolite, wherein the content of the porous deodorant is 0.25 mass% or more based on the total amount of the adhesive composition;
   an antibacterial agent containing a silver ion-supporting zeolite, wherein the content of the antibacterial agent is 0.1 mass% or more based on the total amount of the adhesive composition; and
   an adhesive base.

2. The adhesive composition of claim 1, wherein the content of the porous deodorant is 0.4 mass% or more, and the content of the antibacterial agent is 0.15 to 0.5 mass%.

3. The adhesive composition of any one of claims 1 or 2, which has a softening point higher than 70°C measured by the ring and ball method in accordance with JAI 7-1999.

4. The adhesive composition of any one of claims 1 to 3, wherein the adhesive base contains one or more thermoplastic elastomers selected from the group consisting of a styrene-isoprene-styrene block polymer, a styrene-butadiene-styrene block copolymer, a styrene-ethylene-butylene-styrene block copolymer, and a styrene-ethylene-propylene-styrene block copolymer.

5. A method for producing the adhesive composition of any one of claims 1 to 4, comprising:

   a process where a masterbatch containing the porous deodorant and the antibacterial agent is mixed with an adhesive base component other than the adhesive base component contained in the masterbatch; or
   a process where a masterbatch containing the porous deodorant and a masterbatch containing the antibacterial agent are mixed with an adhesive base component other than the adhesive base component contained in the masterbatches.

6. An object comprising a laminated structure part in which at least two layers are adhered to each other by the adhesive composition of any one of claims 1 to 4.

7. The object of claim 6, which is a body fluid absorbing object having the laminated structure part.

### Patentansprüche

1. Klebstoffzusammensetzung, die Folgendes umfasst:

   ein poröses Deodorant, das einen Zinkionen-tragenden Zeolithen enthält, wobei der Gehalt des porösen Deodorants 0,25 Masse-% oder mehr, bezogen auf die Gesamtmenge der Klebstoffzusammensetzung, beträgt;
   ein antibakterielles Mittel, das einen Silberionen-tragenden Zeolithen enthält, wobei der Gehalt des antibakteriellen Mittels 0,1 Masse-% oder mehr, bezogen auf die Gesamtmenge der Klebstoffzusammensetzung, beträgt; und
   eine Klebstoffbasis.

2. Klebstoffzusammensetzung nach Anspruch 1, wobei der Gehalt des porösen Deodorants 0,4 Masse- % oder mehr und der Gehalt des antibakteriellen Mittels 0,15 bis 0,5 Masse-% beträgt.

3. Klebstoffzusammensetzung nach einem der Ansprüche 1 oder 2, die einen Erweichungspunkt von mehr als 70 °C aufweist, gemessen nach der Ring- und Kugelmethode gemäß JAI 7-1999.

4. Klebstoffzusammensetzung nach einem der Ansprüche 1 bis 3, wobei die Klebstoffbasis ein oder mehrere thermoplastische Elastomere umfasst, die ausgewählt sind aus der Gruppe bestehend aus einem Styrol-Isopren-Styrol-Blockpolymer, einem StyrolButadien-Styrol-Blockcopolymer, einem Styrol-Ethylen-Butylen-Styrol-Blockcopolymer und einem Styrol-Ethylen-Propylen-Styrol-Blockcopolymer.

5. Verfahren zur Herstellung der Klebstoffzusammensetzung nach einem der Ansprüche 1 bis 4, umfassend:

einen Prozess, bei dem ein Masterbatch, das das poröse Deodorant und das antibakterielle Mittel enthält, mit einer anderen Klebstoffbasiskomponente als der im Masterbatch enthaltenen Klebstoffbasiskomponente gemischt wird; oder

einen Prozess, bei dem ein Masterbatch, das das poröse Deodorant enthält, und ein Masterbatch, das das antibakterielle Mittel enthält, mit einer anderen Klebstoffbasiskomponente als der in den Masterbatches enthaltenen Klebstoffbasiskomponente gemischt werden.

6. Artikel mit einem laminierten Strukturteil, bei dem wenigstens zwei Schichten durch die Klebstoffzusammensetzung nach einem der Ansprüche 1 bis 4 miteinander verklebt sind.

7. Artikel nach Anspruch 6, der ein Körperflüssigkeit absorbierender Artikel ist, der das laminierte Strukturteil aufweist.

**Revendications**

1. Composition adhésive comprenant :

un déodorant poreux contenant une zéolite supportant des ions zinc, la teneur du déodorant poreux étant de 0,25 % en masse ou plus par rapport à la quantité totale de la composition adhésive ;
un agent antibactérien contenant une zéolite supportant des ions argent, la teneur de l'agent antibactérien étant de 0,1 % en masse ou plus par rapport à la quantité totale de la composition adhésive ; et
une base adhésive.

2. Composition adhésive selon la revendication 1, dans laquelle la teneur du déodorant poreux est de 0,4 % en masse ou plus, et la teneur de l'agent antibactérien est de 0,15 à 0,5 % en masse.

3. Composition adhésive selon l'une quelconque des revendications 1 ou 2, qui présente un point de ramollissement supérieur à 70 °C mesuré par le procédé de l'anneau et de la bille conformément à la norme JAI 7-1999.

4. Composition adhésive selon l'une quelconque des revendications 1 à 3, dans laquelle la base adhésive contient un ou plusieurs élastomères thermoplastiques choisis dans le groupe constitué par un polymère séquencé styrène-isoprène-styrène, un copolymère séquencé styrène-butadiène-styrène, un copolymère séquencé styrène-éthylène-butylène-styrène, et un copolymère séquencé styrène-éthylène-propylène-styrène.

5. Procédé de production de la composition adhésive selon l'une quelconque des revendications 1 à 4, comprenant :

un processus dans lequel un mélange-maître contenant le déodorant poreux et l'agent antibactérien est mélangé à un composant de base adhésif autre que le composant de base adhésif contenu dans le mélange-maître ; ou
un processus dans lequel un mélange-maître contenant le déodorant poreux et un mélange-maître contenant l'agent antibactérien sont mélangés à un composant de base adhésif autre que le composant de base adhésif contenu dans les mélanges-maîtres.

6. Objet comprenant une partie structurelle stratifiée dans laquelle au moins deux couches sont collées l'une à l'autre par la composition adhésive selon l'une quelconque des revendications 1 à 4.

7. Objet selon la revendication 6, qui est un objet absorbant les fluides corporels comportant la partie structurelle stratifiée.

FIG. 1

Skin-contacting surface

Skin non-contacting surface

**EP 3 831 904 B1**

## REFERENCES CITED IN THE DESCRIPTION

**Patent documents cited in the description**

- JP 6294978 B
- WO 2018131713 A1
- WO 2007099738 A1
- EP 0697212 A2